# EUROPÄISCHE PATENTSCHRIFT

(11) **EP 1 138 335 B1**
(45) Veröffentlichungstag und Bekanntmachung des Hinweises auf die Patenterteilung: **12.10.2005**
(21) Anmeldenummer: 00106835.2
(22) Anmeldetag: 30.03.2000
(51) Int. Cl.: A61L 2/18

(54) **Verfahren zum maschinellen Reinigen und Desinfizieren von Endoskopen**
Method for the mechanised cleaning and disinfection of endoscopes
Procédé de nettoyage et désinfection mécanique d'endoscopes

(43) Veröffentlichungstag der Anmeldung: 04.10.2001
(73) Patentinhaber: CHEMISCHE FABRIK DR. WEIGERT GMBH & CO.KG., 20539 Hamburg (DE)
(72) Erfinder: Tiarks, Petra, 21031 Hamburg (DE); Kamer, Markus, 21029 Hamburg (DE); Dogs, Carsten, 22043 Hamburg (DE); Staffeldt, Jürgen, 21423 Winsen/Luhe (DE)
(74) Vertreter: Glawe, Delfs, Moll

(56) Entgegenhaltungen:
- EP-A- 0 730 024
- EP-A- 1 070 506
- WO-A-00/26334
- DE-A- 19 744 434
- US-A- 4 297 298
- US-A- 5 077 008
- US-A- 5 720 983

## Beschreibung

Die Erfindung betrifft ein Verfahren zum maschinellen Reinigen und Desinfizieren von Endoskopen und/oder deren Teilen, mit den folgenden Schritten:
a) Reinigen der Endoskope mit einer wäßrigen Reinigungslösung,
b) Desinfizieren der Endoskope mit einer wäßrigen Desinfektionsmittellösung.

In der medizinischen Therapie und Diagnostik werden eine Vielzahl von Eingriffen mit Hilfe von Endoskopen durchgeführt. Diese Endoskope werden bei ihrem Einsatz verunreinigt und mit vielerlei Mikroorganismen infiziert.

Die Reinigung und Desinfektion benutzter Endoskope ist schwierig, da Endoskope, insbesondere moderne Glasfiberendoskope, Oberflächen aus einer Vielzahl unterschiedlicher Materialien aufweisen, die teilweise temperaturempfindlich und teilweise korrosionsanfällig sind. Problematisch ist auch das vollständige Reinigen und Desinfizieren der Vielzahl von Hohlräumen in Endoskopen, insbesondere der im Inneren vorhandenen englumigen Kanäle.

Ein eingangs genanntes Verfahren ist aus EP-A-0 268 227 bekannt. Die wäßrige Desinfektionsmittellösung enthält Glutaraldehyd als Desinfektionsmittel. Aldehyde sind umwelttoxisch und können nur mit besonderen Vorsichtsmaßnahmen gehandhabt werden.

Aus US-A-5 077 008 ist es bekannt, medizinische Instrumente mit einer wäßrigen Lösung von Oxidationsmitteln wie beispielsweise Peressigsäure zu desinfizieren. Das geschilderte Verfahren ist aufwendig, da die Instrumente ausschließlich zum Zwecke des Desinfizierens in eine separate Desinfektionseinrichtung per Hand eingelegt werden müssen. Zur Inhibierung der Korrosivität der Peressigsäure wird gelehrt, Molybdate und Chromate zuzusetzen.

EP-A-1 070 506 (Dokument nach Art. 54 (3) EPÜ) offenbart ein maschinelles Desinfizieren von Endoskopen mit einer wäßrigen Lösung, die Peressigsäure, Wasserstoffperoxid sowie Essigsäure enthält und dem Desinfektionsschritt eine manuelle bzw. maschinelle Reinigung vorgeschaltet ist.

Der Erfindung liegt die Aufgabe zugrunde, ein Verfahren der eingangs genannten Art zu schaffen, mit dessen Hilfe ein einfaches, schnelles und wirkungsvolles maschinelles Reinigen und Desinfizieren von Endoskopen bzw. deren Teilen möglich ist. Die Erfindung löst diese Aufgabe dadurch, daß der pH-Wert der Desinfektionsmittellösung zwischen 3 und 5 liegt und die Desinfektionsmittellösung wenigstens ein Peroxid enthält.

Zunächst seien einige im Rahmen der Erfindung verwendete Begriffe erläutert.

Der Begriff "Endoskope und/oder deren Teile" umfaßt alle im Bereich der Diagnostik, Therapie und Chirurgie eingesetzten flexiblen und starren Endoskope, die bei bestimmungsgemäßem Gebrauch verunreinigt bzw. kontaminiert werden können, sowie Teile dieser Instrumente bzw. Apparate.

Das erfindungsgemäße Verfahren ist verwendbar für starre Endoskope (bspw. Laparoskope für die minimalinvasive Chirurgie). Besonders vorteilhaft ist dieses Verfahren bei flexiblen Endoskopen, bspw. Glasfiber-Endoskopen, die in ihren flexiblen Bereichen häufig englumige und schwer zu reinigende Hohlräume aufweisen. Flexibel im Sinne der Erfindung sind Endoskope dann, wenn sie Teile oder Bereiche aufweisen, die sich bei bestimmungsgemäßem Gebrauch verbiegen oder verformen können.

Das Reinigen und Desinfizieren erfolgt erfindungsgemäß maschinell. Dies bedeutet, daß ein Eingriff von Hand beim Reinigen und/oder Desinfizieren nicht erforderlich ist. Insbesondere eignet sich das erfindungsgemäße Verfahren zur Anwendung in üblichen Spülmaschinen für medizinische Instrumente und Apparate, in denen der Reinigungs- und Desinfektionsschritt nacheinander in dem gleichen Spültank ablaufen kann. Diese Spülmaschinen weisen bevorzugt Einrichtungen bzw. Anschlüsse für die Endoskope auf, so daß die jeweils in der Maschine umgewälzte Lösung auch durch die Kanäle der Endoskope gepumpt wird und diese so von innen reinigt, desinfiziert oder spült.

Die Begriffe Reinigungslösung bzw. Desinfektionsmittellösung bezeichnen die anwendungsfertigen entsprechenden wäßrigen Lösungen, die in der Regel durch Verdünnen von Konzentraten mit vorzugsweise enthärtetem oder vollentsalztem Wasser hergestellt werden.

Die Desinfektionsmittellösung enthält erfindungsgemäß wenigstens ein Peroxid. Der Begriff Peroxid bezeichnet im Rahmen der Erfindung jegliche in wäßriger Lösung und saurem Milieu hinreichend stabile Peroxid-Verbindung, die unter den Anwendungsbedingungen durch Sauerstofffreisetzung hinreichend mikrobiozid (desinfizierend) wirkt. Wasserstoffperoxid, organische oder anorganische Peroxide können verwendet werden. Besonders bevorzugt sind Peroxycarbonsäuren wie beispielsweise Peroxyessigsäure, die häufig auch mit dem Trivialnamen Peressigsäure belegt wird.

Die Erfindung hat erkannt, daß peroxidhaltige saure Desinfektionsmittellösungen als wirksame Mikrobiozide zur Desinfektion von Endoskopen in einem üblichen maschinellen Reinigungs- und Desinfektionsverfahren Verwendung finden können, ohne daß es des Zusatzes besonderer Korrosionsinhibitoren oder sonstiger Vorsichtsmaßnahmen zur Verhinderung von Korrosionserscheinungen an Oberflächen der Endoskope bedarf. Dies ist überraschend, da die US-A-5 077 008 bei der Verwendung von Peressigsäure als Desinfektionswirkstoff den Zusatz solcher Korrosionsinhibitoren für zwingend erforderlich hält und zusätzlich lehrt, den pH-Wert der Desinfektionslösung durch Zusatz entsprechender Puffer neutral einzustellen, um so die korrosive Wirkung der Peressigsäure zu minimieren. Die erfindungsgemäße Desinfektionsmittellösung ist daher bevorzugt frei von Korrosionsinhibitoren für Metalle wie insbesondere Aluminium und Stahl, enthält also insbesondere keine korrosionsinhibierenden Stoffe wie beispielsweise Borate, Molybdate, Chromate, Tungsdate, Vanadate und dergleichen.

Abweichend vom Stand der Technik lehrt die vorliegende Erfindung einen sauren pH-Wert, der zwischen 3 und 5 liegt. Die Erfindung hat erkannt, daß Peroxide wie insbesondere Peressigsäure bei diesen pH-Werten eine stärkere mikrobiozide Wirkung entfalten, ohne auf metallische oder sonstige Oberflächen von Endoskopen korrosiv zu wirken. Aufgrund der stärker mikrobioziden Wirkung im sauren Milieu kann auch in einer üblichen Spülmaschine für endoskopische Instrumente eine hinreichende Desinfektion erzielt werden, eines aufwendigen separaten Desinfektionsapparates wie in US-A-5 077 008 bedarf es nicht.

Bei Verwendung des bevorzugten Desinfektionsmittels Peressigsäure enthält die gebrauchsfertige Desinfektionsmittellösung davon vorzugsweise 0,005-1 Gew.-%, weiter vorzugsweise 0,01-0,1 Gew.-%, besonders bevorzugt etwa 0,03 Gew.-%. Weitere Peroxide wie beispielsweise Wasserstoffperoxid können zusätzlich enthalten sein, insbesondere dann, wenn man das Desinfektionsmittelkonzentrat zur Vermeidung einer Explosionsgefahr nicht mit Peressigsäure ansetzt, sondern diese in dem Konzentrat in situ aus Wasserstoffperoxid und Essigsäure herstellt.

Die Temperatur der Desinfektionsmittellösung während des Desinfizierens beträgt bevorzugt 15-60°C, weiter vorzugsweise 20-40°C, insbesondere etwa 25°C. Die Dauer des Desinfizierens liegt bevorzugt zwischen 1-30 min, weiter vorzugsweise 2-15 min, weiter vorzugsweise 3-10 min. Häufig wird eine Desinfektionszeit von etwa 5 min verwendet.

Die der Desinfektion vorgeschaltete Reinigung der Endoskope kann mit einem im Stand der Technik bekannten Reinigungsmittel, das sich für Endoskope eignet, in herkömmlicher Weise durchgeführt werden. Besonders bevorzugt ist die Verwendung schwach saurer, neutraler oder schwach alkalischer Reinigungslösungen, da diese die empfindlichen Endoskopen schonend reinigen und wenig aggressiv sind. Der pH-Wert der anwendungsfertigen Reinigungslösung liegt daher bevorzugt zwischen 6 und 10. Enzymatische Reiniger können erfindungsgemäß verwendet werden. Geeignete enzymatische Reiniger sind beispielsweise beschrieben in EP-A-0 730 024. Ein geeigneter enzymfreier Reiniger ist beschrieben in WO-A-9918184. Die Offenbarung dieser beiden Schriften wird durch Bezugnahme darauf ausdrücklich auch zum Gegenstand der vorliegenden Anmeldung gemacht.

Im Rahmen der Erfindung wird sich an das Reinigen und Desinfizieren in der Regel noch ein Nachspülen bevorzugt mit desinfiziertem, vollentsalztem Wasser anschließen, um die Oberflächen der Endoskope von Restmengen Reinigungs- und/oder Desinfektionsmittel zu befreien. Dieses Nachspülen findet bevorzugt bei einer Temperatur von 15-60°C, weiter vorzugsweise 40-60°C, besonders bevorzugt etwa 55°C statt. Bevorzugt wird für einen Zeitraum von 30 s-10 min, weiter vorzugsweise 1-5 min, besonders bevorzugt etwa 2 min nachgespült. An das Nachspülen schließt sich in der Regel ein Trocknen an.

Bei Bedarf kann vor dem Reinigungsschritt auch ein Vorspülen mit kaltem oder warmen, bevorzugt enthärtetem oder vollentsalztem Wasser durchgeführt werden. Optional kann zwischen Reinigungs- und Desinfektionsschritt ein Zwischenspülschritt mit kaltem oder warmem Wasser (auch bevorzugt enthärtet oder vollentsalzt) durchgeführt werden.

Die Erfindung wird nachfolgend anhand von Beispielen erläutert.

### Beispiel 1

Herstellung eines enzymatischen Reinigungsmittelkonzentrats.

Es wurde ein Reinigungsmittelkonzentrat der folgenden Zusammensetzung zubereitet:

| | |
|---|---|
| Citronensäure | 1,6 Gew.-% |
| NORAMER® ¹⁾ 2000 (handelsüblicher Komplexbildner auf Basis von Carboxylat/Sulfonat-Acrylcopolymeren) | |
| Sulfetal® ²⁾ | 3,8 Gew.-% |
| Na-Cumolsulfonat ³⁾ | 8,0 Gew.-% |
| p-Hydroxybenzoesäure | 0,5 Gew.-% |
| Triethanolamin | 7,5 Gew.-% |
| Esperase 8.0 L⁴⁾ | 2,0 Gew.-% |
| Rest (auf 100 Gew.-%) Wasser | |

| | |
|---|---|
| ¹⁾ Firma NorsoHaas S.A., Verneuil, En Halatte, Frankreich | |
| ²⁾ Gemisch aus Isooctylsulfat-Na- und Amylsulfat-Na-Salz, 38%ig, berechnet auf 100%ige Konzentration | |
| ³⁾40%ig, berechnet als 100%iges Präparat | |
| ⁴⁾Firma Novo Industries, Dänemark | |

Dieses Reinigungsmittelkonzentrat ist offenbart in EP-A-0 730 024.

### Beispiel 2

Herstellung eines enzymfreien Reinigungsmittelkonzentrats.

Es wurde ein Reinigungsmittelkonzentrat der folgenden Zusammensetzung zubereitet:

| | |
|---|---|
| Triethanolamin 85% | 15,0 |
| Hordaphos® MDGB¹⁾ | 1,0 |
| Citronensäure | 1,2 |
| NTA Trinatriumsalz 40%ige wäßrige Lösung | 15,0 |
| Noramer® 2000²⁾ | 0,4 |
| p-Hydroxybenzoesäure methylester | 0,5 |
| N-Acylglutamat | 0,4 |
| Wasser (vollentsalzt) | 66,5 |
| pH-Wert des Konzentrats | 9,4 |
| pH-Wert einer 1%igen wäßrigen Lösung (in VE-Wasser) | 9,9 |

| | |
|---|---|
| ¹⁾ Diester der Phosphorsäure mit Butanol und Ethylenglykol | |
| ²⁾ Komplexbildner auf Basis von Carboxylat/Sulfonat-Acrylcopolymeren, Firma Norso Haas S.A., Verneuil, En Halat-te, Frankreich | |

Die Reinigungsmittelkonzentrate gemäß Beispiel 1 oder 2 werden bevorzugt mit enthärtetem oder vollentsalztem Wasser in einer Konzentration von 0,1-10 Gew.-%, bevorzugt etwa 0,2-2 Gew.-%, weiter vorzugsweise etwa 0,5 Gew.-%, zu einer in Schritt a) des erfindungsgemäßen Verfahrens verwendbaren Reinigungslösung angesetzt.

### Beispiel 3

Es wurde ein Desinfektionsmittelkonzentrat der folgenden Zusammensetzung zubereitet:
- Wasser (vollentsalzt) 0,5%
- Bayhibit AM®¹⁾ 0,1%
- Schwefelsäure (70%) 0,8%
- Essigsäure (99/100%) 10,0%
- Wasserstoffperoxid (30%) 88,6%

¹⁾2-Phosphono-1,2,4-butantricarbonsäure zur Maskierung von Metallionen und Stabilisierung des Peroxids.

In dieser Mischung entsteht Peressigsäure in situ. Nach der Gleichgewichtseinstellung enthält die Mischung etwa 5,7 Gew.-% Peressigsäure und etwa 18 Gew.-% Wasserstoffperoxid.

### Beispiel 4

Dieses Beispiel beschreibt die Durchführung des erfindungsgemäßen Verfahrens.

Benutzte Endoskope werden in einer handelsüblichen Spülmaschine für endoskopische Instrumente und Apparate eingelegt und darin fixiert. Die durchspülbaren Kanäle der Endoskope werden mit entsprechenden Anschlüssen der Spülmaschine verbunden, so daß die jeweils in der Spülmaschine umgewälzte wäßrige Lösung auch durch diese Kanäle gepumpt wird.

Zur Durchführung des Reinigungsschrittes läßt man enthärtetes Wasser in die Spülmaschine einlaufen und erwärmt dieses auf etwa 40°C. Ein Reinigerkonzentrat gemäß Beispiel 1 wird in einer Menge von 0,5 Gew.-% bezogen auf die eingesetzte Wassermenge eindosiert. Die Reinigungsmittellösung wird bei 40°C für 3 min in der Spülmaschine umgewälzt. Anschließend wird die Spülflotte abgelassen, bei Bedarf kann sie vor dem Verbringen in die Kanalisation chemisch oder thermisch desinfiziert werden.

Zur Durchführung des Desinfektionsschrittes läßt man kaltes, enthärtetes Wasser einlaufen und erwärmt dieses auf 25°C. Das Desinfektionsmittelkonzentrat des Beispiels 3 wird in einer Menge von 0,5 Gew.-% bezogen auf die eingesetzte Wassermenge eindosiert und die Desinfektionsflotte während 5 min bei der genannten Temperatur umgewälzt. Der pH-Wert der eingesetzten Desinfektionsflotte liegt bei etwa 4. Die Desinfektionsflotte wird anschließend abgelassen.

Zum Nachspülen wird desinfiziertes, vollentsalztes Wasser eingelassen, auf 55°C erwärmt und für 2 min in der Maschine umgewälzt. Danach wird das Nachspülwasser abgelassen.

Zum Schluß erfolgt eine Trocknung während 3 min bei einer Temperatur von 55°C.

Nach dem Ende des Reinigungs- und Desinfektionsprogramms können die Endoskope gebrauchsfertig aus der Spülmaschine entnommen werden.

## Patentansprüche

1. Verfahren zum maschinellen Reinigen und Desinfizieren von Endoskopen und/oder deren Teilen, mit den Schritten:
a) Reinigen der Endoskope mit einer wäßrigen Reinigungslösung,
b) Desinfizieren der Endoskope mit einer wäßrigen Desinfektionsmittellösung,
**dadurch gekennzeichnet, daß** der pH-Wert der Desinfektionsmittellösung zwischen 3 und 5 liegt und die Desinfektionsmittellösung wenigstens ein Peroxid enthält.

2. Verfahren nach Anspruch 1, **dadurch gekennzeichnet, daß** die Desinfektionsmittellösung wenigstens eine Peroxycarbonsäure enthält.

3. Verfahren nach Anspruch 2, **dadurch gekennzeichnet, daß** die Desinfektionsmittellösung Peroxyessigsäure (Peressigsäure) enthält.

4. Verfahren nach Anspruch 3, **dadurch gekennzeichnet, daß** der Peroxyessigsäuregehalt der Desinfektionsmittellösung 0,005-1 Gew.-%, vorzugsweise 0,01-0,1 Gew.-% beträgt.

5. Verfahren nach einem der Ansprüche 1 bis 4, **dadurch gekennzeichnet, daß** die Temperatur der Desinfektionsmittellösung in Schritt b) 15-60°C, vorzugsweise 20-40°C, weiter vorzugsweise etwa 25°C beträgt.

6. Verfahren nach einem der Ansprüche 1 bis 5, **dadurch gekennzeichnet, daß** die Dauer des Desinfektionsschrittes 1-30 min, vorzugsweise 2-15 min, weiter vorzugsweise 3-10 min beträgt.

7. Verfahren nach einem der Ansprüche 1 bis 6, **dadurch gekennzeichnet, daß** der pH-Wert der Reinigungslösung in Schritt a) zwischen 6 und 10 liegt.

8. Verfahren nach einem der Ansprüche 1 bis 7, **dadurch gekennzeichnet, daß** die Reinigungslösung Enzyme enthält.

## Claims

1. A method for the mechanised cleaning and disinfection of endoscopes and/or parts thereof, having the steps:
a) cleaning of the endoscopes with an aqueous cleaning solution,
b) disinfection of the endoscopes with an aqueous disinfectant solution,
**characterised in that** the pH value of the disinfectant solution is between 3 and 5 and the disinfectant solution contains at least one peroxide.

2. A method according to claim 1, **characterised in that** the disinfectant solution contains at least one peroxycarboxylic acid.

3. A method according to claim 2, **characterised in that** the disinfectant solution contains peroxyacetic acid (peracetic acid).

4. A method according to claim 3, **characterised in that** the peroxyacetic acid content of the disinfectant solution amounts to 0.005 - 1 wt.%, preferably 0.01 - 0.1 wt.%.

5. A method according to any one of claims 1 to 4, **characterised in that** the temperature of the disinfectant solution in step b) amounts to 15 - 60°C, preferably 20 - 40°C, more preferably approximately 25°C.

6. A method according to any one of claims 1 to 5, **characterised in that** the duration of the disinfection step amounts to 1 - 30 min, preferably 2 - 15 min, more preferably 3 - 10 min.

7. A method according to any one of claims 1 to 6, **characterised in that** the pH value of the cleaning solution in step a) lies between 6 and 10.

8. A method according to any one of claims 1 to 7, **characterised in that** the cleaning solution contains enzymes.

## Revendications

1. Procédé pour le nettoyage et la désinfection mécaniques d'endoscopes et/ou de parties de ceux-ci, comprenant les étapes consistant à :
a) nettoyer l'endoscope avec une solution nettoyante aqueuse ;
b) désinfecter l'endoscope avec une solution désinfectante aqueuse ;
**caractérisé en ce que** le pH de la solution désinfectante est compris entre 3 et 5 et **en ce que** la solution désinfectante contient au moins un peroxyde.

2. Procédé selon la revendication 1, **caractérisé en ce que** la solution désinfectante contient au moins un acide carbonique de peroxyde.

3. Procédé selon la revendication 2, **caractérisé en ce que** la solution désinfectante contient un acétate de peroxyde.

4. Procédé selon la revendication 3, **caractérisé en ce que** la teneur en acétate de peroxyde de la solution désinfectante se situe entre 0,005 et 1 % en poids, et de préférence entre 0,01 et 0,1 % en poids.

5. Procédé selon l'une quelconque des revendications 1 à 4, **caractérisé en ce que** la température de la solution désinfectante à l'étape b) se situe entre 15 et 60 °C, de préférence entre 20 et 40 °C, et davantage de préférence à environ 25 °C.

6. Procédé selon l'une quelconque des revendications 1 à 5, **caractérisé en ce que** la durée de l'étape de désinfection se situe entre 1 et 30 minutes, de préférence entre 2 et 15 minutes, et davantage de préférence entre 3 et 10 minutes.

7. Procédé selon l'une quelconque des revendications 1 à 6, **caractérisé en ce que** le pH de la solution nettoyante à l'étape a) est compris entre 6 et 10.

8. Procédé selon l'une quelconque des revendications 1 à 7, **caractérisé en ce que** la solution nettoyante contient des enzymes.
